# EUROPÄISCHE PATENTSCHRIFT

(11) **EP 3 865 497 B1**
(45) Veröffentlichungstag und Bekanntmachung des Hinweises auf die Patenterteilung: **25.03.2026**
(21) Anmeldenummer: 20217295.3
(22) Anmeldetag: 24.12.2020
(51) Int. Cl.: C07H 1/00, C07H 1/06, C07H 3/02, A23L 2/60, A23L 27/30, A23L 29/30

(54) **VERFAHREN ZUR HERSTELLUNG FARBLOSER KOHLENHYDRATE ODER FARBLOSER KOHLENHYDRATKRISTALLE**
METHOD FOR THE PREPARATION OF COLOURLESS CARBOHYDRATES OR COLOURLESS CARBOHYDRATE CRYSTALS
PROCÉDÉ DE FABRICATION DE CARBOHYDRATES INCOLORES OU DE CRISTAUX DE CARBOHYDRATE INCOLORES

(30) Priorität: 12.02.2020 EP 20156820
(43) Veröffentlichungstag der Anmeldung: 18.08.2021
(73) Patentinhaber: SAVANNA Ingredients GmbH & Co. KG, 50189 Elsdorf (DE)
(72) Erfinder: JUNKLEWITZ, Anna, 42119 Wuppertal (DE); MOHR, Stephan, 53879 Euskirchen (DE); BUTZ, Steffen, 52372 Kreuzau (DE); KOCH, Timo, 50189 Elsdorf (DE)
(74) Vertreter: Kutzenberger Wolff & Partner

(56) Entgegenhaltungen:
- EP-A1- 3 006 568
- EP-A1- 3 210 478
- EP-B1- 2 552 241
- EP-B1- 3 006 568
- EP-B1- 3 210 478
- WO-A1-2018/087261
- WO-A2-2018/081557
- US-A- 4 950 332

## Beschreibung

### GEBIET DER ERFINDUNG

Die Erfindung befindet sich auf dem Gebiet der Lebensmitteltechnologie und betrifft ein Verfahren zur Entfärbung von wässrigen Kohlenhydratlösungen, nämlich wässriger Allulose-Zubereitungen.

### TECHNOLOGISCHER HINTERGRUND

Unter die Zuckerersatzstoffe werden die Süßstoffe und die Zuckeralkohole gerechnet. Süßstoffe können auf natürlichem oder synthetischem Weg hergestellt werden. Sie zeichnen sich dadurch aus, dass sie keinen oder nur einen vernachlässigbar geringfügigen Nährwert besitzen, was sie für Menschen, die Diät halten, besonders interessant macht.

Süßstoff scheidet der Körper entweder ganz oder größtenteils unverändert aus. Sie finden sich in vielen Light- und Diätprodukten. Zuckeraustauschstoffe sind Kohlenhydrate, die nur einen geringen Anstieg des Blutzucker- und Insulinspiegels verursachen. Ihre Süßkraft beträgt 40 bis 70 Prozent der Süßkraft des normalen Haushaltszuckers. Der Anteil von Zuckerersatzstoffen in Lebensmitteln und Getränken hat in den letzten Jahren kontinuierlich zugenommen. Im Rahmen der vorliegenden Erfindung soll unter dem Begriff *"niedrigkalorisch"* ein Brennwert von nicht mehr als 10, vorzugsweise nicht mehr als 5 und insbesondere von 1 bis 2 kcal verstanden werden.

Die industriell bedeutsamsten Zuckerersatzstoffe sind Acesulfam K und Aspartam. Es handelt sich hierbei um synthetische Stoffe, die in einigen Studien als krebsgefährdend bezeichnet werden. Diese bisher nicht bestätigten Befunde stärken jedoch den allgemeinen Trend hin zu *"natürlichen"* Zuckerersatzstoffen.

In den letzten Jahren haben Stevioside breite Aufmerksamkeit erlangt, da die darin enthaltenen Einzelstoffe wie Rebaudiosid A eine bis zu 1000fach stärkere Süßkraft als Haushaltszucker besitzen und dabei nichtkalorisch sind. Nachteilig ist jedoch, dass Stevioside allgemein einen harten, metallischen Nachgeschmack aufweisen und sich daher bislang nicht als Zuckerersatzstoffe haben etablieren können.

Diese Lücke könnte geschlossen werden durch die so genannten *"seltenen Zuckern"* wie Tagatose, Cellobiose oder Allulose (die synonym auch als Psicose bezeichnet wird).

Allulose (Psicose) ist ein kalorienarmer Zucker mit ähnlich süßem Geschmack von Zucker. Allulose ist einer von vielen verschiedenen Zuckern, die in der Natur in sehr geringen Mengen vorkommen. Allulose wurde ursprünglich aus Weizen identifiziert und wurde seitdem in bestimmten Früchten wie Jackfrucht, Feigen und Rosinen gefunden. Allulose ist natürlich in kleinen Mengen in einer Vielzahl von süßen Lebensmitteln wie Karamellsauce, Ahornsirup und braunem Zucker enthalten. Allulose wird vom Körper absorbiert, aber nicht metabolisiert, sodass sie nahezu kalorienfrei ist.

Aufgrund des wachsenden Interesses eines großen Teils der Bevölkerung an *"gesunder Ernährung"* und *"gesunder Lebensweise"* allgemein, hat Allulose als kalorienfreier Zucker ein großes Interesse in der Lebensmittelindustrie und in der wissenschaftlichen Community erweckt.

Allulose wird oft in Form von Kristallen kommerzialisiert.

### RELEVANTER STAND DER TECHNIK

H. Itoh et al, Journal of Fermentation Bioengeneering, 80(1), 1995, S. 101-103 veröffentlicht die Herstellung von D-Psicose aus D-Fructose durch immobilisierte D-Tagatose 3-Epimerase.

N. Wagner et al in Organic Process Research Development 2012, 16, S. 323-330 bezieht sich auf praktische Aspekte des integrierten Betriebs von Biotransformation und simulierter Bewegungsbetttrennung (SMB) für die feinchemische Synthese. D-Psicose wird unter Verwendung der D-Tagatose Epimerase-katalysierten Epimerisierung aus D-Fructose hergestellt.

N. Wagner et al, in Chemical Engineering Science 137 (2015) S. 423-435 bezieht sich auf die modellbasierte Kostenoptimierung eines integrierten Prozesses zur enzymatischen Herstellung von Psicose bei erhöhten Temperaturen.

Bosshart et al, in Biotechnology Bioengineering 2016, 113(2), S. 349-58 bezieht sich auf die Produktion der seltenen Zucker D-Psicose und L-Tagatose durch zwei künstlich hergestellte D-Tagatose-Epimerasen.

N. Wagner, et al., in Journal of Chromatography A 2015, 1398, S. 47-56 offenbart ein Verfahren zur wirtschaftlichen Herstellung von d-Psicose durch simulierte Wanderbettchromatographie.

Aus der EP 2552241 B1 ist ein einstufiges Verfahren zur Konzentrierung von wässrigen Allulose-Lösungen bekannt, bei dem man eine übersättigte Zuckerlösung bei einer Temperatur von 60 bis 70 °C in der Lösung eindampft. Das Verfahren erfordert jedoch einen hohen Energieaufwand. Zudem sind hohe Verweilzeiten im Verdampfer erforderlich, was bei den anliegenden Temperaturen Anlass zu Maillard-Reaktionen gibt. Die resultierenden Produkte weisen daher häufig unerwünschte Verfärbungen auf, die eine Vermarktung erschweren.

Das Dokument EP 2 552 241 B1 offenbart ein Verfahren zur Herstellung hochreiner D-Allulose-Kristalle mit einer Reinheit von 98% (w/w) oder mehr und einer Korngröße von MA200 oder mehr, umfassend: Entfernen von Verunreinigungen aus einer D-Allulose-Lösung, um eine gereinigte D-Allulose-Lösung zu erhalten; Konzentrieren der gereinigten D-Allulose-Lösung; Kühlen der konzentrierten D-Allulose-Lösung auf 30°C bis 40°C durch einen Wärmetauscher; Impfen der D-Allulose-Lösung bei 30°C bis 40°C, und anschließende Kristallisation.

Das Dokument US 4 950 332 offenbart ein Verfahren zum Entfärben einer wässrigen Zuckerlösung, die Farbkörper enthält. Das Verfahren umfasst den Schritt des Inkontaktbringens einer wässrigen Zuckerlösung mit einer wirksamen Menge eines adsorbierenden Harzes, um Farbkörper aus der wässrigen Zuckerlösung auf dem adsorbierenden Harz zu adsorbieren.

Die EP 3564250 A2 empfiehlt zur Entfärbung von Verfärbungen von Allulose-Lösungen die Behandlung mit Aktivkohlepulver.

Gegenstand der WO 2018 087261 A1 (PFEIFER&LANGEN) ist ein Verfahren zur Synthese eines Einfachzuckers, vorzugsweise von D-Allulose aus einem Edukt, vorzugsweise von D-Fructose unter heterogener oder homogener Katalyse, die eine chemische und/oder enzymatische Katalyse beinhaltet, wobei die Synthese in mindestens zwei in Reihe geschalteten Reaktoren durchgeführt wird und das aus dem ersten Reaktor austretende Reaktionsprodukt vor dem Eintritt in den zweiten Reaktor einer chromatographischen Trennung unterzogen wird. Vorzugsweise wird die chromatographische Trennung in ein simuliertes Wanderbett integriert.

Die EP 3 006 568 A1**,** WO 2018/081557 und EP 3 210 478 A1 offenbaren weitere Zusammensetzungen, welche Kohlenhydrate enthalten.

### AUFGABE DER ERFINDUNG

Infolge der Temperaturbelastung bei der Konzentrierung flüssiger Allulose-Zubereitungen, die als Intermediate anfallen und entweder als solche in den Handel gelangen (*"Allulosesirup"*) oder einer Kristallisation unterworfen werden, kann es beispielsweise durch Maillard-Reaktion zu Verfärbungen der Kohlenhydrate kommen. Die Endprodukte weisen dann einen bräunlichen Farbton auf, was eine Vermarktung erschwert, weil der Verbraucher dies als Hinweis auf eine mindere Qualität versteht.

Die Aufgabe der vorliegenden Erfindung hat daher darin bestanden, die aus dem Stand der Technik bekannten Entfärbungsverfahren zu verbessern, so dass farblose Kohlenhydrate, insbesondere Allulose, mit ausgezeichneten geschmacklichen Eigenschaften gewonnen werden können.

Eine weitere Aufgabe der vorliegenden Erfindung hat somit darin bestanden, ein Verfahren zur Herstellung von farblosen Allulosekristallen zur Verfügung zu stellen, so dass die erhaltenen Allulosekristalle rieselfähig, leicht wasserlöslich und geschmacklich einwandfrei sind.

### BESCHREIBUNG DER ERFINDUNG

Die Erfindung ist durch die beigefügten Ansprüche definiert.

Die folgende Beschreibung unterliegt dieser Einschränkung. Alle Aspekte und Ausführungsformen, die nicht unter die Ansprüche fallen, sind lediglich Aspekte der vorliegenden Offenbarung und nicht Teil der Erfindung.

Gegenstand der vorliegenden Erfindung ist ein Verfahren zur Herstellung farbloser Allulose oder farbloser Allulosekristalle, umfassend oder bestehend aus den folgenden Schritten:
(a) Bereitstellen einer wässrigen Allulose-Lösung mit einer Reinheit >90%, bezogen auf den gesamten Trockensubstanz-Gehalt der Lösung, wobei die Allulose-Lösung einen Gehalt an Fructose von maximal 10 Gew.-% bezogen auf den gesamten Trockensubstanz-Gehalt der Lösung aufweist;
(b) Konzentrieren der wässrigen Lösung aus Schritt (a) durch einen thermischen Verdampfungsschritt unter Erhalt eines wässrigen Konzentrats, welches einen Trockenmasse-Gehalt von 60 bis 85 Gew.-% aufweist;
(c) Inkontaktbringen des wässrigen Konzentrats aus Schritt (b) mit Adsorptionsmittel unter Erhalt eines entfärbten Konzentrats, wobei mindestens zwei Adsorptionssäulen in Reihe geschaltet sind, die mit unterschiedlichen Adsorptionsmitteln gefüllt sind ausgewählt aus
   (i) Aktivkohlegranulat und
   (ii) organischen synthetischen Harzen bzw. Ionenaustauschern;
(d) optionale Konzentrierung des entfärbten Konzentrats des Schrittes (c);
(e) optionale Kristallisierung des Konzentrats aus Schritt (d) und nachfolgende optionale Trocknung der Kristalle.

Überraschenderweise wurde gefunden, dass Aktivkohle in granulierter Form sowie Adsorberharze nicht nur geeignetere Mittel darstellen, um Zuckerlösungen im Allgemeinen und Allulose-Verdampferkonzentrate im Besonderen in kurzen Zeiten mit hohem Durchsatz zu entfärben, sondern die resultierenden entfärbten Produkte zudem auch noch geschmackliche Vorteile sowie eine erhöhte Lagerstabilität aufweisen.

Im Sinne der Offenbarung kommen als Kohlenhydrate im Schritt (a) des erfindungsgemäßen Verfahrens insbesondere niedrigkalorische Mono- und/oder Disaccharide mit vorzugsweise 6 bis 12 Kohlenstoffatomen in Frage.

Es handelt sich erfindungsgemäß bei der wässrigen Lösung des Schrittes (a) um wässrige Lösungen von Allulose.

Wie bereits erwähnt, weist die wässrige Lösung des Schrittes (a) eine Reinheit >90%, bezogen auf den gesamten TS-Gehalt der Lösung, auf.

Der Trockensubstanz-Gehalt (TS-Gehalt) der wässrigen Lösung des Schrittes (a) liegt im Bereich 8-35%, vorzugsweise 9-30%, bevorzugter 9-28%, bevorzugter 9-26%, bevorzugter 9-25%, und insbesondere bevorzugt im Bereich 20-23%.

In einer bevorzugte Ausführungsform weist die wässrige Lösung des Schrittes (a) eine Reinheit >91% auf, vorzugsweise >92%, vorzugsweise >93%, vorzugsweise >94%, vorzugsweise >95%, vorzugsweise >95%, vorzugsweise >96%, vorzugsweise >97%, vorzugsweise >98% und vorzugsweise >99%, bezogen auf den gesamten TS-Gehalt der Lösung.

In einer bevorzugten Ausführungsform weist die wässrige Lösung des Schrittes (a) eine Reinheit >93% auf, bezogen auf den gesamten Feststoffgehalt der Lösung, wobei die der TS-Gehalt der Lösung im Bereich 9-30% liegt.

Erfindungsgemäß handelt es sich bei der wässrigen Lösung des Schrittes (a) um wässrige Lösungen von Allulose.

Die Herstellung von Psicose/Allulose durch Umlagerung von Fructose in Gegenwart einer Epimerase stellt freien Stand der Technik dar und geht auf Arbeiten der Universität Kagawa aus dem Jahre 1994 zurück. *Ken Izumori* entdeckte dabei, dass D-Tagatose 3-epimerase in der Lage ist, D-Fructose in D-Psicose umzulagern **(**Biosci. Biotechnol. Biochem., 1994, 58:12, 2168-2171**).** Die vorliegende Erfindung umfasst dabei auch jeweils Racemate, Stereoisomeren einzelner Kohlenhydrate sowie Mischungen verschiedener Kohlenhydrate, ohne dass im Folgenden jeweils einzeln noch einmal darauf hingewiesen wird.

Da die wässrige Lösung des Schrittes (a) eine wässrige Allulose-Lösung ist, ist es möglich, dass neben Allulose auch andere Nebenprodukte, die hauptsächlich aus der enzymatischen Umwandlung von Fructose in Psicose stammen, in der Lösung vorhanden sind. Bei diesen Nebenprodukten handelt sich hauptsächlich um Fructose und Salzen.

In einer bevorzugten Ausführungsform des erfindungsgemäßen Verfahrens ist die wässrige Lösung des Schrittes (a) eine Allulose-Lösung, die einen Gehalt an Fructose von maximal etwa 5 Gew.-%, vorzugsweise von maximal etwa 4 Gew.-%, vorzugsweise von maximal etwa 3 Gew.-%, vorzugsweise von maximal etwa 2 Gew.-%, vorzugsweise von maximal etwa 1 Gew.-%, bevorzugt von maximal etwa 0,5 Gew.-%, und besonders bevorzugst von maximal 0,1 Gew.-%, bezogen auf den gesamten TS-Gehalt der Lösung, aufweist.

In einer weiteren bevorzugten Ausführungsform des erfindungsgemäßen Verfahrens ist die wässrige Lösung des Schrittes (a) eine Allulose-Lösung, die einen Gehalt an Salzen von maximal etwa 2 Gew.-%, vorzugsweise von maximal etwa 1 Gew.-%, vorzugsweise von maximal etwa 0,9 Gew.-%, vorzugsweise von maximal etwa 0,8 Gew.-%, vorzugsweise von maximal etwa 0,7 Gew.-%, vorzugsweise von maximal etwa 0,6 Gew.-%, bevorzugt von maximal etwa 0,5 Gew.-%, und besonders bevorzugst von maximal 0,1 Gew.-%, bezogen auf den gesamten TS-Gehalt der Lösung, aufweist.

In einer weiteren bevorzugten Ausführungsform des erfindungsgemäßen Verfahrens ist die wässrige Lösung des Schrittes (a) eine Allulose-Lösung, die eine Reinheit >95% und einen Gehalt an Fructose von maximal 5 Gew.-% und einen Gehalt an Salzen von maximal 0,1 Gew.-% aufweist, jeweils bezogen auf den gesamten TS-Gehalt der Lösung.

Im Schritt (b) des erfindungsgemäßen Verfahrens wird die wässrige Lösung des Schrittes (a) konzentriert, und zwar durch einen thermischen Verdampfungsschritt.

Im Sinne der vorliegenden Erfindung kommen verschiedenen Verdampfern für die Durchführung der Konzentrierungsschrittes (b) in Frage, beispielsweise Dampfkessel, Dünnschichtverdampfer, Fallfilmverdampfer, Kesselverdampfer, Koaxialverdampfer, Naturumlaufverdampfer, Plattenverdampfer oder Zwangsumlaufverdampfer. Diese Einrichtungen sind für den Fachmann bestens bekannt und brauchen daher nicht näher erläutert werden.

Der Verdampfungsprozess findet typisch entweder in einem oder in zwei bis drei in Reihe geschalteten Verdampfern statt; vorzugsweise werden dabei Fallfilmverdampfer oder Plattenverdampfer verwendet.

Mittels des Schrittes (b) des erfindungsgemäßen Verfahrens wird ein wässriges Konzentrat erhalten, welches einen Trockenmasse-Gehalt von 60 bis 85 Gew.-%, vorzugsweise von etwa 65 bis etwa 80 Gew.-%, bevorzugter von etwa 70 bis etwa 80 Gew.-%, und insbesondere bevorzugt von etwa 70 bis etwa 78 Gew.-% aufweist.

Die Verdampfung wird in der Regel bei einer Temperatur von etwa 50 bis etwa 75 °C durchgeführt. Wird der Verfahrensschritt nicht einstufig, sondern in zwei oder drei Stufen durchgeführt, hat es sich aus energetischen Gründen als vorteilhaft erwiesen, bei der Temperaturführung den Bereich zwischen 59 und 71 °C auszulassen, also in den Verdampfern in Temperaturbereichen darüber und darunter zu arbeiten.

Im Schritt (c) des erfindungsgemäßen Verfahrens erfolgt die Entfärbung des wässrigen Konzentrates des Schrittes (b).

Zur Entfärbung werden die wässrigen Konzentrate mit den Entfärbungsmitteln, also entweder den Aktivkohlegranulaten oder den organischen synthetischen Harzen und Ionenaustauschern, über eine ausreichende Zeitdauer in Kontakt gebracht.

Das Aktivkohlegranulat hat dabei vorzugsweise eine Körnung bei der 90 % der Teilchen einen Durchmesser von etwa 0,5 bis etwa 1 mm aufweisen. Besonders bevorzugt ist ein Granulat der Firma Cabot mit einem durchschnittlichen Partikeldurchmesser von etwa 0,65 mm, das unter der Bezeichnung Norit GAC 1240 erhältlich ist.

Die bevorzugten Adsorptionsmittel stellen makroporöse Harze dar, wie sie im Folgenden exemplarisch aufgeführt sind: Lewatit A 365, Lewatit A 8071, Lewatit A 8072, Lewatit A 8072 PLUS, Lewatit A 8073, Lewatit AF 5, Lewatit S 1567, Lewatit S 1568, Lewatit S 1668, Lewatit S 2328, Lewatit S 2568, Lewatit S 2568 H, Lewatit S 4228, Lewatit S 4268, Lewatit S 4328, Lewatit S 4468, Lewatit S 4528, Lewatit S 5128, Lewatit S 5221, Lewatit S 5228, Lewatit S 5328, Lewatit S 5528, Lewatit S 6268, Lewatit S 6368, Lewatit S 6368 A, Lewatit S 6368 A OH, Lewatit S 6368 A SO4, Lewatit S 6368 Sulfat, Lewatit S 7468, Lewatit S 7968, Lewatit S 8107, Lewatit S 8223, Lewatit S 8227, Lewatit S 8227 Ca, Lewatit S 8227 Mg, Lewatit S 8229, Lewatit S 8229 Plus / Ag, Lewatit S 8229 PLUS X, Lewatit S 8528, Lewatit S 9167, Dowex HCR-S/S, Dowex HCR-S/S FF, Dowex HCR-W2, Dowex-HGR-NG, Dowex Marathon C 10, Dowex Monosphere C 350, Dowex Monosphere C 400, Dowex Marathon C , Dowex Marathon MSC, Dowex Marathon 1200, Dowex Marathon 1300 H, Dowex 88, Dowex 88 MB, Dowex Monosphere 88 MB, Dowex Monosphere C 600 B, Dowex 66, Dowex Monosphere 66, Dowex 22, Dowex Optipore SD 2, Dowex Optipore L493, Dowex Optipore V493, Dowex Optipore V502, XUS 43565.01, Dowex Optipore V323, Dowex N 406, Dowex Marathon A, Dowex Marathon A LB, Dowex Marathon A2, Dowex Marathon MSA, Dowex Marathon 11, Dowex Marathon MSA - , Dowex NSR-1, Dowex-PSR-2, Dowex Marathon 4200 CI, Treverlite IXC100, Treverlite IXC110, Treverlite IXC200, Treverlite IXC201, Treverlite IXC210, Treverlite IXC230, Treverlite IXC330, Treverlite IXA300, Treverlite IXA310, Treverlite IXA510, Treverlite IXA600, Treverlite IXA610, Treverlite IXA620, Treverlite IXA710, Treverlite CHE710, Treverlite CHE720, Treverlite CHE730, Treverlite XS100200, Treverlite XS103500, Treverlite XS106500, Treverlite XS122400. Bei den entsprechenden Entfärberharzen handelt es sich um kommerzielle Produkte, die von den Herstellern Lanxess, Dow und Chemra bezogen werden können. Besonders gute Ergebnisse wurden dabei mit den Ionenaustauschern erzielt, die als Matrix Styrol-Divinylbenzol-Copolymere, quernetzte Polystyrene, quervernetztes Phenol-Formaldehyd-Kondensat oder Mischungen von diesen aufweisen. Als kationische Gruppen kommen vor allem tertiäre Amine und quaternäre Amine, als anionische Gruppen Sulfonsäuren und Sulfonate in Betracht.

Erfindungsgemäß erfolgt die Entfärbung in Adsorptionssäulen.

Es hat sich als vorteilhaft herausgestellt, die Entfärbung kontinuierlich in zwei Adsorptionssäulen durchzuführen, die im Wechsel betrieben werden.

Demzufolge wird in einer weiteren bevorzugten Ausführungsform die Entfärbung kontinuierlich in zwei Adsorptionssäulen durchgeführt, die im Wechsel betrieben werden.

Die Entfärberharze bzw. das Aktivkohlegranulat können im wässrigen Konzentrat aus Schritt (b) eingerührt und nach einer Verweilzeit von einigen Minuten bis zu einer Stunde abfiltriert werden. Es hat sich aber als vorteilhaft erwiesen, die Entfärbung kontinuierlich über Adsorptionssäulen durchzuführen, die mit dem Granulat oder den synthetischen Harzen gefüllt sind. Über Durchsatz und Länge der Säule lässt sich die Verweilzeit so steuern, dass am Ende der Säule(n) schließlich eine Lösung mit gewünschter Farbreduktion austritt. Es empfiehlt sich zwei Reihen Säulen parallel zu fahren, so dass eine im Wechsel jeweils gereinigt werden kann.

Erfindungsgemäß schaltet man zwei oder mehr Säulen in Reihe und füllt sie mit unterschiedlichen Adsorptionsmitteln, insbesondere ein Adsorberharz in der ersten und ein Aktivkohlegranulat in der zweiten Säule.

Gegenüber Aktivkohlepulver weist das Granulat zwar den Nachteil auf, dass es eine geringere spezifische Oberfläche besitzt, dies wird jedoch dadurch mehr als ausgeglichen, dass das Granulat in Säulen angeordnet werden kann, die von dem wässrigen Konzentrat aus Schritt (b) durchströmt werden können. Die Granulierung der Aktivkohle sowie die Form der Harzpartikel führt gegenüber dem Einsatz von pulverförmigen Agenzien zu einem geringeren Druckverlust beim Durchströmen einer gepackten Säule und daher zu einem niedrigeren Energieverbrauch. Zudem hat sich der Einsatz von gepackten Säulen dadurch als vorteilhaft erwiesen, dass das Harzmaterial beziehungsweise die Aktivkohlegranulate durch die Säulen zurückgehalten und nicht mehr in einem zusätzlichen Schritt von der zu entfärbenden Lösung abgetrennt werden müssen. Werden Harze eingesetzt, hat dies weiter den Vorteil, dass diese in einfacher Weise chemisch regeneriert werden können. Die Aktivkohle muss hingegen nach dem Erreichen der Entfärbungskapazität aufwändig ausgebrannt werden. Sowohl beim Einsatz von Aktivkohlegranulat als auch synthetischen Harzen fällt zudem kein Feinstaub an.

Das im Schritt (c) gewonnene entfärbte Konzentrat kann als solches kommerzialisiert ("Sirup") werden. Typischerweise weist das entfärbte Konzentrat des Schrittes (c) ("Sirup") einen Feststoffgehalt von 55 bis 90 Gew.-% (im Fall von erfindungsgemäßen Allulose-Konzentraten von etwa 65 bis etwa 85 Gew.-%) auf.

In einer bevorzugten Ausführungsform weist das Konzentrat (Allulose-Konzentrat) des Schrittes (c) ("Sirup") einen TS-Gehalt ≥70%, eine Reinheit bezogen auf den TS-Gehalt ≥70%, einen Fructose-Gehalt ≤10% bezogen auf den TS-Gehalt, und einen Salz-Gehalt von ≤0,04 % bezogen auf den TS-Gehalt, sowie einen ICUMSA Farbwert ≤35 ([IE420] bestimmt nach GS 9(1/2/3-8 (2011)) auf.

Das entfärbte Konzentrat des Schritts (c) kann optional aber auch zur Kristallisation eingesetzt werden.

Falls eine Kristallisierung erfolgen soll, wird das Konzentrat des Schrittes (c) optional bis auf einen TS-Gehalt von mindestens 60 Gew.-% in einem Verdampfer konzentriert und anschließend im übersättigten Bereich, nahe des Sättigungspunkts, mit Impfkristallen versetzt. Die Übersättigung wird anschließend durch Verdampfung oder langsames Abkühlen (0,1-1K/h) aufrechterhalten bis sich ein Kristallgehalt von etwa 30 bis etwa 50 Gew.-% eingestellt hat.

Im Sinne der vorliegenden Erfindung kommen verschiedenen Verdampfern für die Durchführung des optionalen Konzentrierungsschritts in Frage, beispielsweise Dampfkessel, Dünnschichtverdampfer, Fallfilmverdampfer, Kesselverdampfer, Koaxialverdampfer, Naturumlaufverdampfer, Plattenverdampfer oder Zwangsumlaufverdampfer. Diese Einrichtungen sind für den Fachmann bestens bekannt und brauchen daher nicht näher erläutert werden.

Wenn eine Kristallisation erfolgt, werden die gewonnenen farblosen Kristalle einer Trocknung unterworfen.

Im Sinne der vorliegenden Erfindung kommen verschiedene Trocknungsanlagen für die Durchführung der Trocknung gemäß Schritt (e) in Frage, beispielsweise Trommeltrockner, Bandtrockner, Schachttrockner, Konustrockner, Konvektionstrockner, Vibrationsfließbetttrockner, Wirbelschichttrockner, Rohrbündeltrockner, Dünnschichttrockner, Scheibentrockner, Vakuumtrockner, Kontakttrockner und Mikrowellentrockner. Diese Trocknungsanlagen sind für den Fachmann bestens bekannt und brauchen daher nicht näher erläutert werden.

Jedoch hat sich als besonders vorteilhaft die Durchführung der Trocknung gemäß Schritt (e) in einer Trocknungsanlage ausgewählt aus der Gruppe bestehend aus Wirbelschichttrockner (kontinuierlich), Vakuumtrockner, Bandtrockner und Dünnschichttrockner, insbesondere Wirbelschichttrockner und Vakuumtrockner, erwiesen.

Der Wassergehalt der gewonnenen Kristalle liegt im Bereich von etwa 0,1 bis etwa 0,5 Gew.-%, vorzugsweise von etwa 0,1 bis etwa 0,3 Gew.-%, bevorzugt von etwa 0,15 bis 0,25 Gew.-%.

Ein weiterer Gegenstand der vorliegenden Erfindung ist die Verwendung der mittels des erfindungsgemäßen Verfahrens gewonnenen farblosen Konzentrate oder Kristalle, nämlich farblosen Alllulosekonzentrate oder farblosen Allulosekristakle, zur Herstellung von Lebensmitteln.

### BEISPIELE

Die vorliegende Erfindung wird unter Bezugnahme auf die nachstehenden Beispiele leichter zu verstehen sein. Die Beispiele, die nicht unter den Schutzbereich der Ansprüche fallen, werden als Referenzbeispiele angegeben.

Jedoch dienen diese Beispiele lediglich zur Veranschaulichung der Erfindung und können nicht als einschränkend in Bezug auf den Schutzbereich der Erfindung ausgelegt werden.

### BEISPIEL 1 (ERFINDUNGSGEMÄSS)

Es wurde eine wässrige Allulose-Lösung mit einem TS-Gehalt von 23 Gew.-% und einer Reinheit von 98%, einem Gehalt an Fructose von 1 Gew.-%, und einem Gehalt an Salzen von 0,5 Gew.-%., jeweils bezogen auf den gesamten TS-Gehalt der Lösung bereitgestellt und mittels eines Plattenverdampfers aufkonzentriert. Dadurch wurde eine wässrige Allulose-Lösung mit einem TS-Gehalt von 71 Gew.-% erhalten. Die Stammlösung wies einen ICUMSA Farbwert (bestimmt nach GS 9(1/2/3-8 (2011) von ca. 9.000 IE auf und wurde anschließend durch Zugabe von Wasser auf einen Farbwert von 500 bzw. 1370 IE eingestellt.

Zwei beheizbare Glaskolonnen jeweils mit einer Länge von 100 cm und einem Durchmesser von 5 cm wurden mit einer Schüttung verschiedener Adsorptionsmitteln versehen, in Reihe geschaltet und mit einem Durchsatz von 25 L/h mit dem Feed beaufschlagt. Der Farbwert wurde jeweils nach dem Verlassen der Kolonnen photometrisch bestimmt. Die Änderung der Farbwerte gegen die Standzeit des Adsorptionsmittels sind in **Tabelle 1** wiedergegeben:

**Tabelle 1A**

| Entfärbung im System Lewatit S 5221/Norit GAC 1240 | | | | | | | | |
|---|---|---|---|---|---|---|---|---|
| | **Kolonne I: Adsorberharz** | | | | **Kolonne Kolonne II: Aktivkohlegranulat** | | | |
| Standzeit [h] | 2 | 4 | 6 | 8 | 2 | 4 | 6 | 8 |
| Farbzahl Feed [IE] | 500 | 500 | 500 | 500 | 50 | 110 | 150 | 175 |
| Farbzahl Produkt [IE] | 50 | 110 | 150 | 175 | 25 **(P1)** | 65 | 90 | 95 |

**Tabelle 1B**

| Entfärbung im System Lewatit S 5221/Norit GAC 1240 | | | | | | | | |
|---|---|---|---|---|---|---|---|---|
| | **Kolonne I: Adsorberhar** | | | | **Kolonne II: Aktivkohlegranulat** | | | |
| Standzeit [h] | 2 | 4 | 6 | 8 | 2 | 4 | 6 | 8 |
| Farbzahl Feed [IE] | 1.370 | 1.370 | 1.370 | 1.370 | 200 | 240 | 290 | 380 |
| Farbzahl Produkt [IE] | 200 | 240 | 290 | 380 | 100 | 110 | 140 | 170 |

Nach Verlassen der ersten Kolonne wurde auch hier in beiden Fällen ein Produkt erhalten, dessen Farbzahl auf 10 % des Ausgangswertes vermindert worden war. Über eine Standzeit von 8 Stunden stieg der Wert bis auf etwa 20 % des Ausgangsfarbwertes an. Mit der zweiten Kolonne ("Polisher") konnte der Farbwert jeweils noch einmal um 50 % gesenkt werden.

### BEISPIEL 2 (Referenzbeispiel)

Eine wässrige Fructose-Lösung (20 Gew.-%) wurde bereitgestellt. Die Lösung wies eine Reinheit von 98% und einen Gehalt an Salzen von 0,5 Gew.-%., jeweils bezogen auf den gesamten TS-Gehalt der Lösung auf. Die Lösung wurde mittels eines Plattenverdampfers aufkonzentriert. Dadurch wurde eine wässrige Fructose-Lösung mit einem TS-Gehalt von 60 Gew.-% erhalten. Die Stammlösung wies einen ICUMSA Farbwert (bestimmt nach GS 9(1/2/3-8 (2011) von ca. 8.600 IE auf und wurde anschließend durch Zugabe von Wasser auf einen Farbwert von 500 bzw. 1370 IE eingestellt.

Zwei beheizbare Glaskolonnen jeweils mit einer Länge von 100 cm und einem Durchmesser von 5 cm wurden mit einer Schüttung verschiedener Adsorptionsmitteln versehen, in Reihe geschaltet und mit einem Durchsatz von 25 L/h mit dem Feed beaufschlagt. Der Farbwert wurde jeweils nach dem Verlassen der Kolonnen photometrisch bestimmt. Die Änderung der Farbwerte gegen die Standzeit des Adsorptionsmittels sind in **Tabelle 2** wiedergegeben:

**Tabelle 2A**

| Entfärbung im System Dowex Monosphere 88 MB/Norit GAC 1240 | | | | | | | | |
|---|---|---|---|---|---|---|---|---|
| | **Kolonne I: Adsorberharz** | | | | **Kolonne II: Aktivkohlegranulat** | | | |
| Standzeit [h] | 2 | 4 | 6 | 8 | 2 | 4 | 6 | 8 |
| Farbzahl Feed [IE] | 500 | 500 | 500 | 500 | 60 | 120 | 140 | 180 |
| Farbzahl Produkt [IE] | 60 | 120 | 140 | 180 | 30 | 70 | 85 | 90 |

**Tabelle 2B**

| Entfärbung im System Dowex Monosphere 88 MB/Norit GAC 1240 | | | | | | | | |
|---|---|---|---|---|---|---|---|---|
| | **Kolonne I: Adsorberharz** | | | | **Kolonne II: Aktivkohlegranulat** | | | |
| Standzeit [h] | 2 | 4 | 6 | 8 | 2 | 4 | 6 | 8 |
| Farbzahl Feed [IE] | 1.370 | 1.370 | 1.370 | 1.370 | 220 | 250 | 300 | 400 |
| Farbzahl Produkt [IE] | 220 | 250 | 300 | 400 | 110 | 120 | 150 | 160 |

Nach Verlassen der ersten Kolonne wurde auch hier in beiden Fällen ein Produkt erhalten, dessen Farbzahl auf 10 % des Ausgangswertes vermindert worden war. Über eine Standzeit von 8 Stunden stieg der Wert bis auf etwa 20 % des Ausgangsfarbwertes an. Mit der zweiten Kolonne ("Polisher") konnte der Farbwert jeweils noch einmal um 50 % gesenkt werden.

### EVALUIERUNG DER KONZENTRATE ("SIRUPS")

Der Sirup V1 wurde mittels Konzentration der wässrigen Allulose-Lösung (60 Gew.-%) des Beispiels 1 bis auf einen TS-Gehalt von 75% hergestellt. Andererseits wurde der Sirup 1 mittels Konzentration des Zwischenproduktes P1 der Tabelle 1 bis auf einen TS-Gehalt von 75% hergestellt.

V1 und Sirup 1 wurden für 7 Monaten bei Raumtemperatur gelagert (unter Licht und Hitze geschützt). Nach Lagerung wurden V1 und Sirup 1 von einem Panel bestehend aus fünf erfahrenen und geschulten Testern in Hinblick auf die optischen und geschmacklichen Eigenschaften evaluiert (3= ausgeprägt, 2= vorhanden, 1= nicht festzustellen). Die Ergebnisse sind in der Tabelle 3 zusammengefasst.

**Tabelle 1**

| **Beispiele** | **Verdunkelung** | **Metallischer Geschmack** |
|---|---|---|
| Sirup 1 | x | 1 |
| Sirup V1 | √ | 3 |
| x= nicht festzustellen; √ = vorhanden | | |

Die experimentellen Daten zeigen, dass die mittels des erfindungsgemäßen Verfahrens hergestellten Sirupe verbesserte Ergebnisse hinsichtlich Optik und Geschmack zeigen.

## Patentansprüche

1. Verfahren zur Herstellung farbloser Allulose oder farbloser Allulosekristalle, umfassend oder bestehend aus den folgenden Schritten:
(a) Bereitstellen einer wässrigen Allulose-Lösung mit einer Reinheit >90%, bezogen auf den gesamten Trockensubstanz-Gehalt der Lösung, wobei die Allulose-Lösung einen Gehalt an Fructose von maximal 10 Gew.-% bezogen auf den gesamten Trockensubstanz-Gehalt der Lösung aufweist;
(b) Konzentrieren der wässrigen Lösung aus Schritt (a) durch einen thermischen Verdampfungsschritt unter Erhalt eines wässrigen Konzentrats, welches einen Trockenmasse-Gehalt von 60 bis 85 Gew.-% aufweist;
(c) Inkontaktbringen des wässrigen Konzentrats aus Schritt (b) mit Adsorptionsmittel unter Erhalt eines entfärbten Konzentrats, wobei mindestens zwei Adsorptionssäulen in Reihe geschaltet sind, die mit unterschiedlichen Adsorptionsmitteln gefüllt sind ausgewählt aus
(i) Aktivkohlegranulat und
(ii) organischen synthetischen Harzen bzw. Ionenaustauschern;
(d) optionale Konzentrierung des entfärbten Konzentrats des Schrittes (c);
(e) optionale Kristallisierung des Konzentrats aus Schritt (d) und nachfolgende optionale Trocknung der Kristalle.

2. Verfahren nach Anspruch 1, **dadurch gekennzeichnet, dass** die wässrige Allulose-Lösung einen Gehalt an Salzen von maximal 2 Gew.-% bezogen auf den gesamten Trockensubstanz-Gehalt der Lösung aufweist.

3. Verfahren nach einem der vorhergehenden Ansprüche, **dadurch gekennzeichnet, dass** die wässrige Allulose-Lösung einen Gehalt an Salzen von maximal 1 Gew.-% bezogen auf den gesamten Trockensubstanz-Gehalt der Lösung aufweist.

4. Verfahren nach einem der vorhergehenden Ansprüche, **dadurch gekennzeichnet, dass** die wässrige Allulose-Lösung einen Gehalt an Salzen von maximal 0,7 Gew.-% bezogen auf den gesamten Trockensubstanz-Gehalt der Lösung aufweist.

5. Verfahren nach einem der vorhergehenden Ansprüche, **dadurch gekennzeichnet, dass** die wässrige Allulose-Lösung einen Gehalt an Salzen von maximal 0,5 Gew.-% bezogen auf den gesamten Trockensubstanz-Gehalt der Lösung aufweist.

6. Verfahren nach einem der vorhergehenden Ansprüche, **dadurch gekennzeichnet, dass** die wässrige Allulose-Lösung einen Gehalt an Salzen von maximal 0,1 Gew.-% bezogen auf den gesamten Trockensubstanz-Gehalt der Lösung aufweist.

7. Verfahren nach einem der vorhergehenden Ansprüche, **dadurch gekennzeichnet, dass** die wässrige Allulose-Lösung eine Reinheit >95% und einen Gehalt an Fructose von maximal 5 Gew.-% und einen Gehalt an Salzen von maximal 0,1 Gew.-% aufweist, jeweils bezogen auf den gesamten Trockensubstanz-Gehalt der Lösung.

8. Verfahren nach einem der vorhergehenden Ansprüche, **dadurch gekennzeichnet, dass** das wässrige Konzentrat des Schrittes (b) einen Trockenmasse-Gehalt von 65 bis 80 Gew.-% aufweist.

9. Verfahren nach einem der vorhergehenden Ansprüche, **dadurch gekennzeichnet, dass** das wässrige Konzentrat des Schrittes (b) einen Trockenmasse-Gehalt von 70 bis 80 Gew.-% aufweist.

10. Verfahren nach einem der vorhergehenden Ansprüche, **dadurch gekennzeichnet, dass** das wässrige Konzentrat des Schrittes (b) einen Trockenmasse-Gehalt von 70 bis 78 Gew.-% aufweist.

11. Verfahren nach einem der vorhergehenden Ansprüche, **dadurch gekennzeichnet, dass** die Verdampfung des Schritts (b) bei einer Temperatur von 50 bis 75 °C durchgeführt wird.

12. Verfahren nach einem der vorhergehenden Ansprüche, **dadurch gekennzeichnet,** die Verdampfung in einem oder in zwei bis drei in Reihe geschalteten Verdampfern durchgeführt wird.

13. Verfahren nach einem der vorhergehenden Ansprüche, **dadurch gekennzeichnet, dass** man die Verdampfung in zwei oder drei Stufen durchführt und bei der Temperaturführung den Bereich zwischen 59 und 71 °C auslässt.

14. Verfahren nach einem der vorhergehenden Ansprüche, **dadurch gekennzeichnet, dass** von den zwei in Reihe geschalteten Adsorptionssäulen die erste mit einem organischen synthetischen Harz und die andere mit Aktivkohlegranulat gefüllt ist.

15. Verfahren nach einem der vorhergehenden Ansprüche, **dadurch gekennzeichnet, dass** man organische synthetische Harze bzw. Ionenaustauscher einsetzt, die
(i) als Matrix Styrol-Divinylbenzol-Copolymere, quernetzte Polystyrene, quervernetztes Phenol-Formaldehyd-Kondensat oder Mischungen von diesen, und/oder
(ii) als kationische Gruppen tertiäre Amine und/oder quaternäre Amine, und/oder
(iii) als anionische Gruppen Sulfonsäuren und/oder Sulfonate aufweisen.

## Claims

1. Method for producing colorless allulose or colorless allulose crystals, comprising or consisting of the following steps:
(a) providing an aqueous allulose solution with a purity >90%, based on the total dry matter content of the solution, wherein the allulose solution has a fructose content of at most 10% by weight, based on the total dry matter content of the solution;
(b) concentrating the aqueous solution from step (a) by means of a thermal evaporation step to obtain an aqueous concentrate having a dry matter content of 60 to 85% by weight;
(c) bringing the aqueous concentrate from step (b) into contact with adsorbents to obtain a decolorized concentrate, wherein at least two adsorption columns are connected in series, which are filled with different adsorbents selected from
(i) activated carbon granules and
(ii) organic synthetic resins or ion exchangers;
(d) optional concentration of the decolorized concentrate from step (c);
(e) optional crystallization of the concentrate from step (d) and subsequent optional drying of the crystals.

2. Method according to claim 1, **characterized in that** the aqueous allulose solution has a salt content of at most 2% by weight based on the total dry matter content of the solution.

3. Method according to one of the preceding claims, **characterized in that** the aqueous allulose solution has a salt content of at most 1% by weight based on the total dry matter content of the solution.

4. Method according to one of the preceding claims, **characterized in that** the aqueous allulose solution has a salt content of at most 0.7% by weight based on the total dry matter content of the solution.

5. Method according to one of the preceding claims, **characterized in that** the aqueous allulose solution has a salt content of at most 0.5% by weight based on the total dry matter content of the solution.

6. Method according to one of the preceding claims, **characterized in that** the aqueous allulose solution has a salt content of at most 0.1% by weight based on the total dry matter content of the solution.

7. Method according to one of the preceding claims, **characterized in that** the aqueous allulose solution has a purity >95% and a fructose content of at most 5% by weight and a salt content of at most 0.1% by weight, in each case based on the total dry matter content of the solution.

8. Method according to one of the preceding claims, **characterized in that** the aqueous concentrate of step (b) has a dry matter content of 65 to 80% by weight.

9. Method according to one of the preceding claims, **characterized in that** the aqueous concentrate of step (b) has a dry matter content of 70 to 80% by weight.

10. Method according to one of the preceding claims, **characterized in that** the aqueous concentrate of step (b) has a dry matter content of 70 to 78% by weight.

11. Method according to one of the preceding claims, **characterized in that** the evaporation in step (b) is carried out at a temperature of 50 to 75 °C.

12. Method according to one of the preceding claims, **characterized in that** the evaporation is carried out in one or in two to three evaporators connected in series.

13. Method according to one of the preceding claims, **characterized in that** the evaporation is carried out in two or three stages and the temperature range between 59 and 71 °C is omitted during temperature control.

14. Method according to one of the preceding claims, **characterized in that** of the two adsorption columns connected in series, the first is filled with an organic synthetic resin and the other with activated carbon granules.

15. Method according to one of the preceding claims, **characterized in that** organic synthetic resins or ion exchangers are used which contain
(i) as a matrix, styrene-divinylbenzene copolymers, cross-linked polystyrenes, cross-linked phenol-formaldehyde condensate or mixtures thereof, and/or
(ii) as cationic groups, tertiary amines and/or quaternary amines, and/or
(iii) as anionic groups, sulfonic acids and/or sulfonates.

## Revendications

1. Procédé de production d'allulose incolore ou de cristaux d'allulose incolores, comprenant ou consistant en les étapes suivantes :
(a) fournir une solution aqueuse d'allulose d'une pureté > 90 % par rapport à la teneur totale en matière sèche de la solution, la solution d'allulose ayant une teneur en fructose maximale de 10 % en poids par rapport à la teneur totale en matière sèche de la solution;
(b) concentrer la solution aqueuse de l'étape (a) par une étape d'évaporation thermique pour obtenir un concentré aqueux ayant une teneur en matière sèche de 60 à 85 % en poids;
(c) mise en contact du concentré aqueux de l'étape (b) avec un adsorbant pour obtenir un concentré décoloré, au moins deux colonnes d'adsorption étant montées en série, qui sont remplies de différents adsorbants choisis parmi
(i) des granulés de charbon actif et
(ii) des résines synthétiques organiques ou des échangeurs d'ions ;
(d) concentration facultative du concentré décoloré de l'étape (c) ;
(e) cristallisation facultative du concentré issu de l'étape (d) et séchage facultatif ultérieur des cristaux.

2. Procédé selon la revendication 1, **caractérisé en ce que** la solution aqueuse d'allulose présente une teneur en sels maximale de 2 % en poids par rapport à la teneur totale en matière sèche de la solution.

3. Procédé selon l'une des revendications précédentes, **caractérisé en ce que** la solution aqueuse d'allulose présente une teneur en sels maximale de 1 % en poids par rapport à la teneur totale en matière sèche de la solution.

4. Procédé selon l'une des revendications précédentes, **caractérisé en ce que** la solution aqueuse d'allulose présente une teneur en sels maximale de 0,7 % en poids par rapport à la teneur totale en matière sèche de la solution.

5. Procédé selon l'une des revendications précédentes, **caractérisé en ce que** la solution aqueuse d'allulose présente une teneur en sels maximale de 0,5 % en poids par rapport à la teneur totale en matière sèche de la solution.

6. Procédé selon l'une des revendications précédentes, **caractérisé en ce que** la solution aqueuse d'allulose présente une teneur en sels maximale de 0,1 % en poids par rapport à la teneur totale en matière sèche de la solution.

7. Procédé selon l'une des revendications précédentes, **caractérisé en ce que** la solution aqueuse d'allulose présente une pureté > 95 % et une teneur en fructose maximale de 5 % en poids et une teneur en sels maximale de 0,1 % en poids, chacune par rapport à la teneur totale en matière sèche de la solution.

8. Procédé selon l'une des revendications précédentes, **caractérisé en ce que** le concentré aqueux de l'étape (b) présente une teneur en matière sèche comprise entre 65 et 80 % en poids.

9. Procédé selon l'une des revendications précédentes, **caractérisé en ce que** le concentré aqueux de l'étape (b) présente une teneur en matière sèche comprise entre 70 et 80 % en poids.

10. Procédé selon l'une des revendications précédentes, **caractérisé en ce que** le concentré aqueux de l'étape (b) présente une teneur en matière sèche comprise entre 70 et 78 % en poids.

11. Procédé selon l'une des revendications précédentes, **caractérisé en ce que** l'évaporation de l'étape (b) est effectuée à une température comprise entre 50 et 75 °C.

12. Procédé selon l'une des revendications précédentes, **caractérisé en ce que** l'évaporation est effectuée dans un ou deux à trois évaporateurs montés en série.

13. Procédé selon l'une des revendications précédentes, **caractérisé en ce que** l'on effectue l'évaporation en deux ou trois étapes et que l'on omet la plage comprise entre 59 et 71 °C lors du contrôle de la température.

14. Procédé selon l'une des revendications précédentes, **caractérisé en ce que**, parmi les deux colonnes d'adsorption montées en série, la première est remplie d'une résine synthétique organique et l'autre de granulés de charbon actif.

15. Procédé selon l'une des revendications précédentes, **caractérisé en ce que** l'on utilise des résines synthétiques organiques ou des échangeurs d'ions qui
(i) comme matrice, des copolymères styrène-divinylbenzène, du polystyrène réticulé, un condensat phénol-formaldéhyde réticulé ou des mélanges de ceux-ci, et/ou
(ii) comme groupes cationiques, des amines tertiaires et/ou des amines quaternaires, et/ou
(iii) comme groupes anioniques des acides sulfoniques et/ou des sulfonates.
